# EUROPEAN PATENT SPECIFICATION

(11) **EP 1 485 098 B1**
(45) Date of publication and mention of the grant of the patent: **03.01.2007**
(21) Application number: 03706615.6
(22) Date of filing: 13.03.2003
(51) Int. Cl.: A61K 31/5025, A61K 9/14, A61K 47/38, A61K 47/10, A61K 47/20, A61P 29/00

(54) **PHARMACEUTICAL COMPOSITIONS COMPRISING 2-(4-ETHOXY-PHENYL)-3-(4-METHANESULFONYL-PHENYL)-PYRAZOLO¬1,5-B|PYRIDAZINE IN NANOPARTICULATE FORM**
PHARMAZEUTISCHE ZUBEREITUNGEN ENTHALTEND NANOPARTIKEL VON 2-(4-ETHOXY-PHENYL)-3-(4-METHANSULPHONYL-PHENYL)-PYRAZOLO¬1,5-B|PYRIDAZIN
COMPOSITIONS PHARMACEUTIQUES CONTENANT 2-(4-ETHOXY-PHENYL)-3-(4-METHANESULFONYL-PHENYL)-PYRAZOLO'1,5-BIPYRIDAZINE SOUS FORME NANOPARTICULAIRE

(30) Priority: 15.03.2002 GB 0206200
(43) Date of publication of application: 15.12.2004
(73) Proprietor: GLAXO GROUP LIMITED, Greenford, Middlesex UB6 ONN (GB)
(72) Inventor: APPLEBY, Jonathan, GlaxoSmithKline, Harlow, Essex CM19 5AW (GB); HILL, Martin Rolfe, GlaxoSmithKline, Harlow, Essex CM19 5AW (GB); HOLLAND, Simon Joseph, GlaxoSmithKline, Harlow, Essex CM19 5AW (GB); PEARSON, Stephanie Lynn, GlaxoSmithKline, Harlow, Essex CM19 5AW (GB)
(74) Representative: Cooke, Tracey
(86) International application number: PCT/EP2003/002698
(87) International publication number: WO 2003/077920

(56) References cited:
- WO-A-01/41760
- WO-A-02/00196

## Description

The present invention relates to certain novel pharmaceutical compositions comprising a selective cyclooxygenase-2 inhibitor, processes for their preparation and to the use of such compositions in the manufacture of medicaments for the treatment of cyclooxygenase - 2 mediated diseases.

WO99/12930 (Glaxo Group Limited) discloses 2-(4-ethoxy-phenyl)-3-(4-methanesulfonylphenyl)-pyrazolo[1,5-b]pyridazine (I) as a potent and selective inhibitor of cyclooxygenase-2 (COX-2).

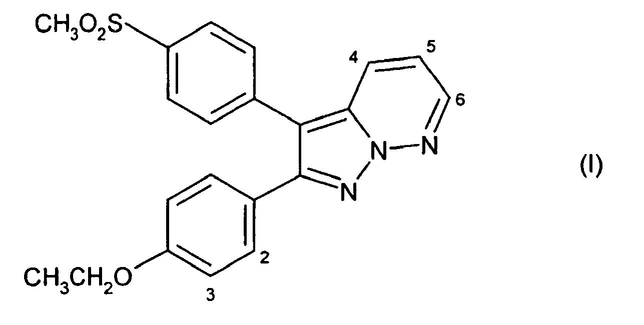

Dissolution testing is a well established technique for obtaining a correlation between in vitro and in vivo data in relation to compound bioavailability. Preliminary dissolution experiments employing compound (I) in micronised form predicted it to be poorly bioavailable. As a result attempts were made to improve the bioavailability of compound (I). WO01/41760 (Pharmacia Corporation) discloses that the bioavailability of selective COX-2 inhibitory drugs of low water solubility may be enhanced by reducing the drug particle size, such that a substantial proportion are smaller than 1 µm. However, dissolution experiments using a pharmaceutical composition comprising compound (I), wherein the drug was present in nanoparticulate form, still resulted in a poor dissolution profile, thus predicting this pharmaceutical composition to be poorly bioavailable in man. Further dissolution experiments indicated that the bioavailability of compound (I) could be enhanced by co-formulation with hydroxypropylmethylcellulose-acetyl succinate (HPMC-AS) in an amorphous dispersion. Moreover a 1:1 ratio of compound (I) and HPMC-AS was found to be optimal.

As a result a pharmaceutical composition comprising a 1:1 ratio of compound (I) and hydroxypropylmethylcellulose-acetyl succinate (HPMC-AS) as an amorphous dispersion has been administered to man at 35mg o.d. The resulting pharmacokinetic parameters such as the maximum blood serum concentration of the drug (Cₘₐₓ), the time to achieve the maximum blood serum concentration of the drug (Tₘₐₓ) and the exposure of the volunteer to the drug as measured by the area under the plasma concentration versus time curve (AUC) were all conducive to the further development of compound (I). However, unacceptably high inter-subject variability was seen for these parameters.

The problem of high inter-subject variability for the parameters Cₘₐₓ, Tₘₐₓ and AUC may be solved by providing a pharmaceutical composition comprising compound (1), wherein the drug is present in nanoparticulate form.

Accordingly, in a first aspect the invention thus provides a pharmaceutical composition comprising a compound of formula (I) 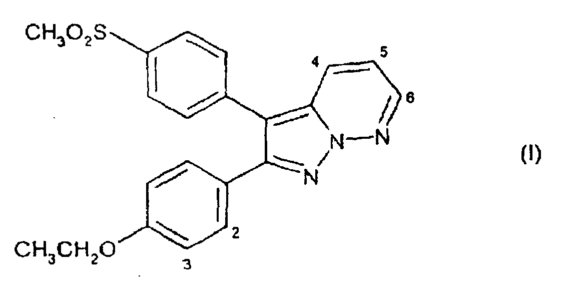 and pharmaceutically acceptable salts thereof, in which the compound is present in solid particles with a median size by volume in the range 0.4 to 1.8 µm, in admixture with one or more pharmaceutically acceptable carriers or excipients, which further comprises HPMC present in less than 15% w/w.

Surprisingly, we have now found that a pharmaceutical composition as defined hereinabove results in significantly reduced inter-subject variability for both Cₘₐₓ, Tₘₐₓ and AUC when dosed in human volunteers. Furthermore, and contrary to expectation, this pharmaceutical composition resulted in a pharmacokinetic profile in man with a shorter Tₘₐₓ, a higher Cₘₐₓ and a higher AUC in comparison with the pharmacokinetic profile for the composition comprising a 1:1 ratio of compound (I) and hydroxypropylmethylcellulose-acetyl succinate (HPMC-AS) as an amorphous dispersion. Such a pharmacokinetic profile is particularly beneficial for the treatment of acute pain disorders where early and rapid relief from the pain or other symptoms is desired.

For the purposes of the present invention "nanoparticulate" is defined as solid particles with a median size by volume [D(v,0.5)] in the range 0.4 to 1.8 µm.

In aspect of the invention compound (I) is present in solid particles with a D(v,0.5) in the range 0.4 to 1.5 µm.

In another aspect of the invention compound (I) is present in solid particles with a D(v,0.5) in the range 0.45 to 1.05 µm.

In another aspect of the invention compound (I) is present in solid particles with a D(v,0.5) of 0.8 µm.

The particle size of the solid particles of compound (I) may be determined by laser diffraction. A suitable machine for determining particle size by laser diffraction is a Sympatec laser diffraction unit, using an HELOS optical bench fitted with a QUIXEL dispersion unit. As used herein, the median size by volume [D(v,0.5)] of the particles is determined by laser diffraction, as defined above.

Numerous processes for the synthesis of solid particles in nanoparticulate form are known. Typically these processes involve a milling process, preferably a wet milling process in the presence of a surface modifying agent that inhibits aggregation and/or crystal growth of the nanoparticles once created. Alternatively these processes may involve a precipitation process, preferably a process of precipitation in an aqueous medium from a solution of the drug in a non-aqueous solvent.

Accordingly, in a further aspect, the present invention provides a process for preparing compound (I) in nanoparticulate form as hereinbefore defined, which process comprises milling or precipitation.

Representative processes for the preparation of solid particles in nanoparticulate form are described in the patents and publications listed below.
U.S. Patent No. 4,826,689 to Violanto & Fischer
U. S. Patent No. 5,145,684 to Liversidge et al
U.S Patent No. 5,298,262 to Na & Rajagopalan
U.S. Patent No. 5,302,401 Liversidge et al
U.S. Patent No. 5,336,507 to Na & Rajagopalan
U.S. Patent No. 5,340,564 to Illig & Sarpotdar
U.S. Patent No. 5,346,702 to Na Rajagopalan
U.S. Patent No. 5,352,459 to Hollister et al
U.S. Patent No. 5,354,560 to Lovrecich
U.S. Patent No. 5,384,124 to Courteille et al
U.S. Patent No. 5,429,824 to June
U.S. Patent No. 5,503,723 to Ruddy et al
U.S. Patent No. 5,510 118 to Bosch et al
U.S. Patent No. 5,518 to Bruno et al
U.S. Patent No. 5,518,738 to Eickhoff et al
U.S. Patent No. 5,534,270 to De Castro
U.S. Patent No. 5,536,508 to Canal et al
U.S. Patent No. 5,552,160 to Liversidge et al
U.S. Patent No. 5,560,931 to Eickhoff et al
U.S. Patent No. 5,560,932 to Bagchi et al
U.S. Patent No. 5,565,188 to Wong et al
U.S. Patent No. 5,571,536 to Eickhoff et al
U.S. Patent No. 5,573,783 to Desieno & Stetsko
U.S Patent No. 5,580,579 to Ruddy et al
U.S. Patent No 5,585,108 to Ruddy et al
U.S. Patent No. 5,587,143 to Wong
U.S. Patent No. 5,591456 to Franson et al
U.S. Patent No. 5,622,938 to Wong
U.S. Patent No 5,662,883 to Bagchi et al
U.S. Patent No. 5,665,331 to Bagchi et al
U.S Patent No. 5,718,919 to Ruddy et al
U.S. Patent No. 5,747,001 to Wiedmann et al
International Patent Publication No WO93/25190
International Patent Publication No. WO96/24336
International Patent Publication No. WO 97/14407
International Patent Publication No. WO 98/35666
International Patent Publication No. WO 99/65469
International Patent Publication No. WO 00/18374
International Patent Publication No. WO 00/27369
International Patent Publication No. WO 00/30615 and
International Patent Publication No. WO 01/41760.

Such processes may be readily adapted for the preparation of compound (I) in nanoparticulate form. Such processes form a further aspect of the invention.

The process of the present invention preferably uses a wet milling step carried out in a mill such as a dispersion mill in order to produce a nanoparticulate form of the compound. The present invention may be put into practice using a conventional wet milling technique, such as that described in Lachman *et al.,* The Theory and Practice of Industrial Pharmacy, Chapter 2, "Milling" p.45 (1986).

In a further refinement, PCT/EP01/07085 (SmithKline Beecham plc) describes a wet milling procedure using a mill in which at least some of the surfaces are made of nylon (polyamide) comprising one or more internal lubricants, for use in the preparation of solid particles of a drug substance in nanoparticulate form.

In another aspect the present invention provides a process for preparing compound (I) in nanoparticulate form comprising wet milling a suspension of compound (I) in a mill having at least one chamber and agitation means, said chamber(s) and/or said agitation means comprising a lubricated nylon, as described in PCT/EP01/07085.

The suspension of compound (I) for use in the wet milling is typically a liquid suspension of the coarse compound in a liquid medium. By "suspension" is meant that the compound is essentially insoluble in the liquid medium. Representative liquid media include an aqueous medium. Using the process of the present invention the average particle size of coarse compound (I) may be up to 1 mm in diameter. This advantageously avoids the need to pre-process the compound.

In a further aspect of the invention the aqueous medium to be subjected to the milling comprises compound (I) present in from about 1% to about 40% w/w, preferably from about 10% to about 30% w/w, more preferably about 20% w/w.

The aqueous medium may further comprise one or more pharmaceutically acceptable watersoluble carriers which are suitable for steric stabilisation and the subsequent processing of compound (I) after milling to a pharmaceutical composition, e.g. by spray drying.
Pharmaceutically acceptable excipients most suitable for steric stabilisation and spray-drying are surfactants such as poloxamers, sodium lauryl sulphate and polysorbates etc; stabilisers such as celluloses e.g. hydroxypropylmethyl cellulose; and carriers such as carbohydrates e.g. mannitol.

In a further aspect of the invention the aqueous medium to be subjected to the milling may further comprise hydroxypropylmethyl cellulose (HPMC) present in from about 0.1 to about 10% w/w, preferably in about 5% w/w in the aqueous medium to be subjected to the milling.

In a further aspect of the invention the aqueous medium to be subjected to the milling may further comprise hydroxypropylmethyl cellulose (HPMC) present in about 3% w/w or 1% w/w.

In a further aspect of the invention the aqueous medium to be subjected to the milling may further comprise mannitol present in from about 1 to about 15% w/w, preferably in about 10% w/w, in the aqueous medium to be subjected to the milling.

In a further aspect of the invention the aqueous medium to be subjected to the milling may further comprise sodium lauryl sulphate present in about 0.2% w/w.

The process of the present invention may comprise the subsequent step of drying compound (I) to yield a powder.

Accordingly, in a further aspect, the present invention provides a process for preparing a pharmaceutical composition as hereinbefore defined, which process comprises producing compound (I) in nanoparticulate form optionally followed by drying to yield a powder.

By "drying" is meant the removal of any water or other liquid vehicle used during the process to keep compound (I) in liquid suspension or solution. This drying step may be any process for drying known in the art, including freeze drying, spray granulation or spray drying. Of these methods spray drying is particularly preferred. All of these techniques are well known in the art. Spray drying/fluid bed granulation of milled compositions is carried out most suitably using a spray dryer such as a Mobile Minor Spray Dryer [Niro, Denmark], or a fluid bed drier, such as those manufactured by Glatt, Germany.

In a further aspect the invention provides a pharmaceutical composition as hereinbefore defined, in the form of a dried powder, obtainable by wet milling solid particles of compound (1) followed by spray-drying the resultant suspension.

Preferably, the pharmaceutical composition as hereinbefore defined, further comprises HPMC present in the range 0.1 to 10% w/w, more preferably in about 5% w/w.

Preferably, the pharmaceutical composition as hereinbefore defined, further comprises HPMC present in about 3% w/w or 8% w/w.

Preferably the pharmaceutical composition as hereinbefore defined, in the form of a dried powder, further comprises mannitol present in less than 30% w/w, preferably in the range 1 to 15% w/w, more preferably in about 10% w/w.

Preferably, the pharmaceutical composition as hereinbefore defined, in the form of a dried powder, further comprises mannitol present in the range 30 to 45% w/w, more preferably in about 34% w/w or 43% w/w.

Preferably, the pharmaceutical composition as hereinbefore defined, in the form of a dried powder, further comprises sodium lauryl sulphate present in about 0.6% w/w.

Preferably the pharmaceutical composition as hereinbefore defined, in the form of a dried powder, further comprises HPMC present in less than 15% w/w, preferably in the range 0.1 to 10% w/w, and mannitol present in less than 30% w/w, preferably in the range 1 to 15% w/w.

Preferably the pharmaceutical composition as hereinbefore defined, in the form of a dried powder, further comprises HPMC present in about 3% w/w, mannitol present in the range 30 to 45% w/w, more preferably in about 34% w/w, and sodium lauryl sulphate present in about 0.6% w/w.

The solid particles of compound (I) obtainable by wet milling, optionally followed by the step of spray-drying, according to the present invention, may be presented in a variety of finished formulations including, for instance, tablets, for example swallow tablets, dispersible tablets and chewable tablets; in capsules; aqueous syrups and sachets. These may be prepared by combining the pharmaceutical composition of the present invention with excipients conventionally used in such formulations such as disintegrants, diluents, lubricants, wetting agents, binding agents, flavoring agents, sweeteners, colouring agents, preservatives, suspending agents, coating agents and fillers, and further processing into finished formulations. Thus, in a further aspect, pharmaceutical formulations of the present invention comprise pharmaceutical compositions as hereinbefore defined, optionally together with one or more excipients such as disintegrants, diluents, lubricants, wetting agents, binding agents, flavoring agents, sweeteners, colouring agents, preservatives, suspending agents, coating agents and fillers.

Representative disintegrants for use in the instant invention illustratively include maize-starch and rice starch, cross-linked N-vinyl-2-pyrrolidinone, sodium starch glycollate, croscarmellose sodium, micrcrystalline or microfine cellulose, low substitued hydroxypropylcellulose (i.e. cellulose partially substitued with 2-hydroxypropyl groups e.g. less than 25% substituted) cross-linked sodium carboxymethylcellulose, swellable ion exchange resins, formaldehyde-casein or alignates.

Representative lubricants for use in the instant invention illustratively include a long chain fatty acid, such as stearic acid, or salts thereof, such as magnesium stearate.

Representative fillers for use in the instant invention illustratively include silicon dioxide, microcrystalline cellulose, dicalcium phosphate, lactose, sorbitol, cacium carbonate or magnesium carbonate.

In another aspect the invention provides a pharmaceutical composition comprising compound (I) in a form which results in a pharmacokinetic profile in a healthy male volunteer study wherein the median Tₘₐₓ is in the range 0.75 to 1.25 hours, the median Cₘₐₓ is in the range 130 to 170 ng/mL and the AUC (last) is in the range 800 to 900 ng/mL.h.

In another aspect the invention is directed to the use of a pharmaceutical composition comprising compound (I) or a pharmaceutically acceptable salt thereof in which the compound is present in solid particles in nanoparticulate form for the manufacture of a medicament for the treatment of a condition which is mediated by COX-2.

The example that follows illustrates the invention but does not limit the invention in any way.

### Example 1 (Pharmaceutical Composition 2)

A 1 kg batch of an aqueous suspension containing 20% w/w of 2-(4-ethoxy-phenyl)-3-(4-methanesulfonyl-phenyl)-pyrazolo[1,5-b]pyridazine and 5% w/w of hydroxypropylmethylcellulose was passed through a Dena DM-100 bead mill. The single 100ml chamber fabricated from Nylacast Nylube was used in a recirculation configuration with the chamber containing 85% by volume of yttrium stabilised zirconium oxide beads (Tosoh, Japan). The batch was processed using four different bead sizes in sequence: 1 mm diameter bead sample, 0.65mm diameter bead sample, 0.4mm diameter bead sample, 0.3mm diameter bead sample. The batch was processed for one hour using each bead sample. The yield was 81.3%. To the finely milled suspension was added 10% w/w mannitol and the resulting suspension subsequently spray-dried to yield Pharmaceutical Composition 2.

Grinding media contamination levels in the spray-dried powder (Pharmaceutical Composition 2) were 7ppm zirconium (Zr) and <1 ppm yttrium (Y).

The product had a median particle size of 1.01 microns as measured by laser diffraction size analysis using a Sympatec laser diffraction unit, with a HELOS optical bench fitted with a QUIXEL dispersion unit.

### Example 2

A 5 kg batch of an aqueous suspension containing 20% w/w of 2-(4-ethoxy-phenyl)-3-(4-methanesulfonyl-phenyl)-pyrazolo[1,5-b]pyridazine, 0.2% w/w of sodium lauryl sulphate and 1% w/w of hydroxypropylmethylcellulose was passed through a Drais Cosmo 5 bead mill. The single 500ml chamber fabricated from Sustaplast Nylon 6G was used in a recirculation configuration with the chamber containing 570 mL of yttrium stabilised zirconium oxide beads (Tosoh, Japan). The batch was processed using two different bead sizes in sequence: 0.8 mm diameter bead sample and a 0.3 mm diameter bead sample. The batch was processed for 23 minutes for the larger bead size and 80 minutes for the smaller bead size. The yield was 90%. To the finely milled suspension was added 10% w/w mannitol and the resulting suspension subsequently spray-dried to yield Example 2.

The product had a median particle size of 0.8 microns as measured by laser diffraction size analysis using a Sympatec laser diffraction unit, with a HELOS optical bench fitted with a QUIXEL dispersion unit.

### Study 1

A randomised, open label, crossover comparison between single oral doses of 35mg of each of two pharmaceutical compositions of 2-(4-ethoxy-phenyl)-3-(4-methanesulfonyl-phenyl)-pyrazolo[1,5-b]pyridazine was conducted in 24 healthy male volunteers. Single oral doses of 35mg of each of two pharmaceutical compositions of 2-(4-ethoxy-phenyl)-3-(4-methanesulfonylphenyl)-pyrazolo[1,5-b]pyridazine were administered to fasted volunteers. Seven day intervals separated each dose. The pharmacokinetic characteristics of each pharmaceutical composition were determined over a 48 hour time period.

Pharmaceutical Composition 1 - Amorphous spray-dried pharmaceutical composition comprising 2-(4-ethoxy-phenyl)-3-(4-methanesulfonyl-phenyl)-pyrazolo[1,5-b]pyridazine and hydroxypropylmethylcellulose-acetyl succinate in a 1:1 ratio produced by conventional spray-drying techniques.

Pharmaceutical Composition 2 - as for Example 1 above

**Table 1: Summary of Median Serum Derived Pharmacokinetic Parameters for Pharmaceutical Compositions 1 and 2 of 2-(4-Ethoxy-phenyl)-3-(4-methanesulfonyl-phenyl)-pyrazolo[1,5-b]pyridazine from a Healthy Male Volunteer Study of 24 Subjects.**

| | AUC last (ng/mL.h) | Tₘₐₓ (h) | Cₘₐₓ (ng/mL) |
|---|---|---|---|
| Pharmaceutical Composition 1 | 492.16 | 1.75 | 72.39 |
| Pharmaceutical Composition 2 | 840.13 | 1 | 152.33 |

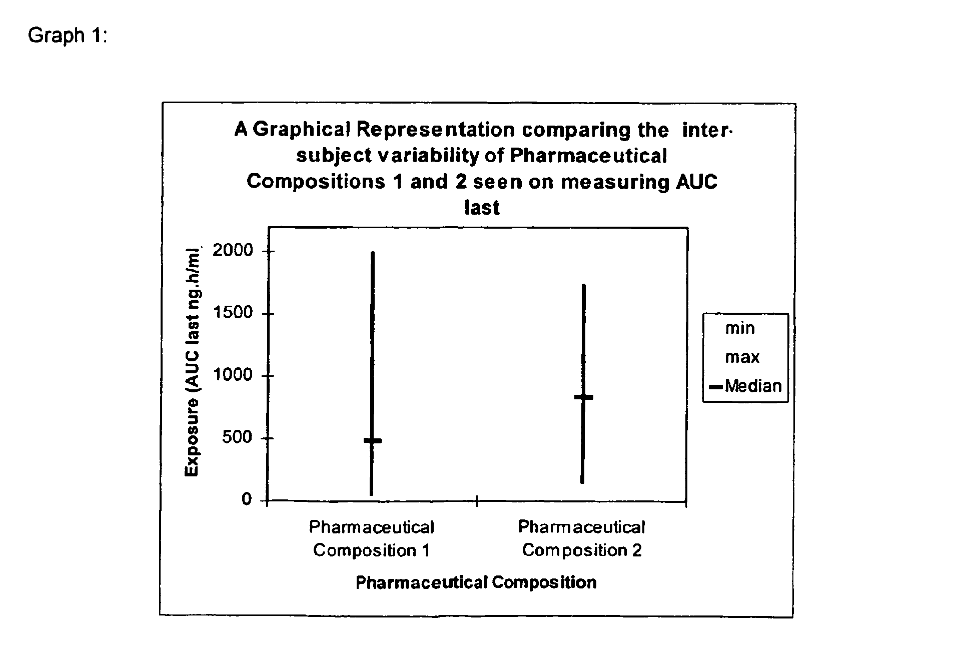

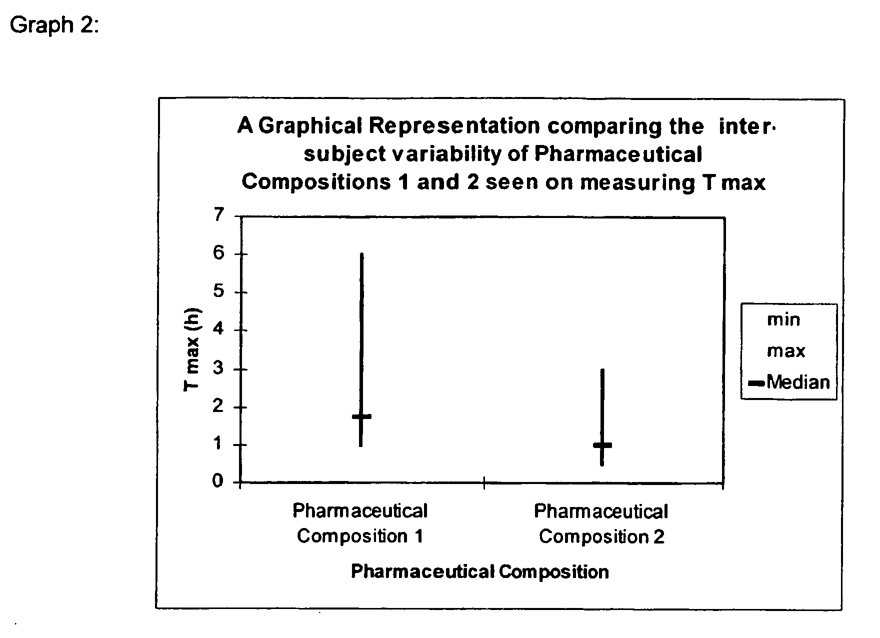

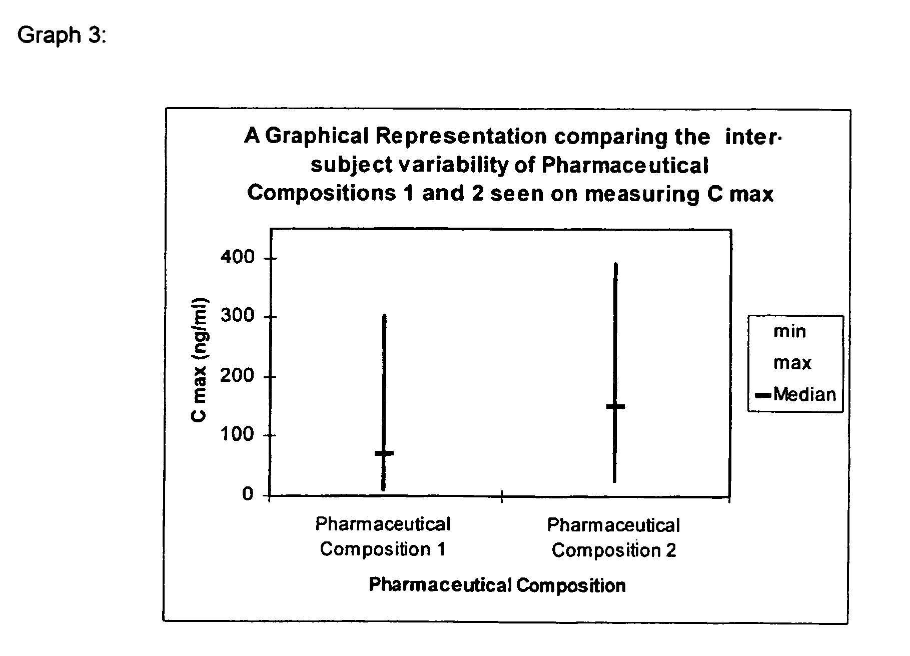

## Claims

1. A pharmaceutical composition comprising compound (1) 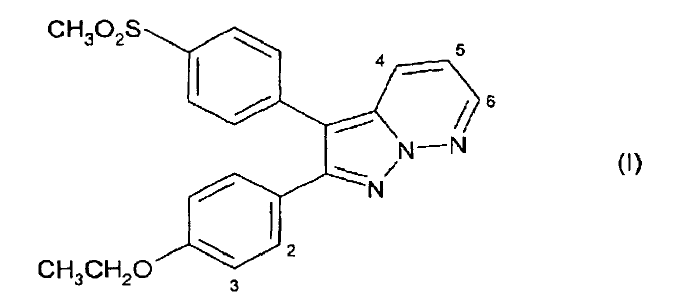 and pharmaceutically acceptable salts thereof, in which the compound is present in solid particles with a median size by volume in the range 0.4 to 1.8µm*,* in admixture with one or more pharmaceutically acceptable carriers or excipients, which further comprises hydroxypropylmethylcellulose (HPMC) present in less than 15% w/w.

2. A pharmaceutical composition as claimed in claim 1 which further comprises mannitol present in the range 30 to 45% w/w.

3. A pharmaceutical composition as claimed in any of claim 1 or claim 2 which further comprises sodium lauryl sulphate present in about 0.6% w/w.

4. A process for preparing a pharmaceutical composition according to any of claims 1 to 3 comprising wet milling a suspension of compound (1) in a mill having at least one chamber and agitation means, said chamber(s) and/or said agitation means comprising a lubricated nylon.

5. A pharmaceutical composition as defined in any of claims 1 to 3 for use in human or veterinary medicine.

6. The use of a pharmaceutical composition as defined in any of claims 1 to 3 for the manufacture of a medicament for the treatment of a condition which is mediated by COX-2.

## Patentansprüche

1. Pharmazeutische Zusammensetzung, umfassend Verbindung (I): 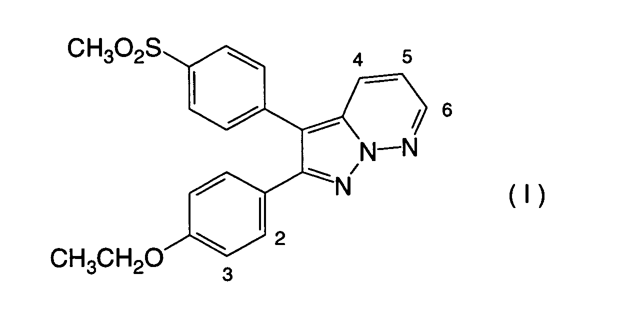 und pharmazeutisch annehmbare Salze davon, in denen die Verbindung in festen Partikeln mit einer mittleren Volumengrösse im Bereich von 0,4 bis 1,8 µm vorliegt, in Mischung mit einem oder mehreren pharmazeutisch annehmbaren Trägern oder Exzipienten, die weiter Hydroxypropylmethylcellulose (HMPC) umfasst, die zu weniger als 15 Gew.% vorhanden ist.

2. Pharmazeutische Zusammensetzung wie in Anspruch 1 beansprucht, die weiter Mannit im Bereich von 30 bis 45 Gew.% umfasst.

3. Pharmazeutische Zusammensetzung wie in irgendeinem der Ansprüche 1 oder 2 beansprucht, die weiter Natriumlaurylsulfat umfasst, das zu etwa 0,6 Gew.% vorhanden ist.

4. Verfahren zur Herstellung einer pharmazeutischen Zusammensetzung gemäss irgendeinem der Ansprüche 1 bis 3, umfassend das Nassmahlen einer Suspension der Verbindung (I) in einer Mühle, die mindestens eine Kammer und Rührvorrichtung aufweist, wobei diese Kammer(n) und/oder diese Rührvorrichtung ein geschmiertes Nylon umfassen.

5. Pharmazeutische Zusammensetzung wie in irgendeinem der Ansprüche 1 bis 3 beansprucht, zur Verwendung in der Human- oder Veterinärmedizin.

6. Verwendung einer pharmazeutischen Zusammensetzung, wie in irgendeinem der Ansprüche 1 bis 3 definiert, zur Herstellung eines Medikaments zur Behandlung eines Zustands, der durch COX-2 vermittelt wird.

## Revendications

1. Composition pharmaceutique comprenant le composé (I) : 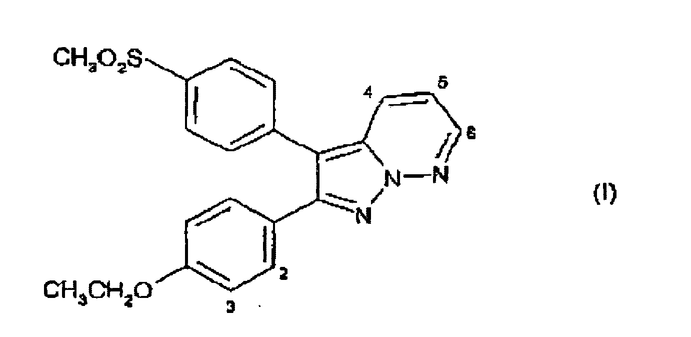 et les sels pharmaceutiquement acceptables de celui-ci, dans laquelle le composé est présent sous la forme de particules solides ayant une taille moyenne en volume allant de 0,4 à 1,8 µm, dans un mélange avec un ou plusieurs supports ou excipients pharmaceutiquement acceptables, qui comprend en outre de l'hydroxypropylméthylcellulose (HPMC) présente dans moins de 15 % p/p.

2. Composition pharmaceutique selon la revendication 1, qu i comprend en outre du mannitol présent dans 30 à 45 % p/p.

3. Composition pharmaceutique selon l'une quelconque des revendications 1 ou 2, qui comprend en outre du laurylsulfate de sodium présent dans environ 0,6 % p/p.

4. Procédé pour préparer une compos ition pharmaceutique selon l'une quelconque des revendications 1 à 3, comprenant l'étape consistant à broyer par voie humide une suspension du composé (I) dans un broyeur ayant au moins un compartiment et un moyen d'agitation, le (s)dit(s) compartiment (s) e t/ou le(s)dit(s) moyen (s) d'agitation comprenant un nylon lubrifié.

5. Composition pharm aceutique selon l'une quelconque des revendications 1 à 3, pour une utilisation en médecine humaine ou vétérinaire.

6. Utilisation d'une composition pharm aceutique selon l'une quelconque des revendications 1 à 3, pour la fabrication d'un médicament pour le traitement d'un état qui est induit par COX-2.
